(19) Europäisches Patentamt
European Patent Office
Office européen des brevets

(11) **EP 3 487 556 B1**

(12) **EUROPÄISCHE PATENTSCHRIFT**

(45) Veröffentlichungstag und Bekanntmachung des Hinweises auf die Patenterteilung:
**06.04.2022 Patentblatt 2022/14**

(21) Anmeldenummer: **17749605.6**

(22) Anmeldetag: **19.07.2017**

(51) Internationale Patentklassifikation (IPC):
**A61M 1/36** (2006.01)

(52) Gemeinsame Patentklassifikation (CPC):
**A61M 1/3639;** A61M 1/3609; A61M 2205/14;
A61M 2205/16; A61M 2205/3331; A61M 2230/207;
A61M 2230/30

(86) Internationale Anmeldenummer:
**PCT/EP2017/000880**

(87) Internationale Veröffentlichungsnummer:
**WO 2018/015017 (25.01.2018 Gazette 2018/04)**

(54) **DIALYSEGERÄT ZUR KORREKTUR DES BLUTFLUSSWERTES**

DIALYSIS DEVICE FOR CORRECTING THE BLOOD FLOW VALUE

APPAREIL DE DIALYSE DE CORRECTION D'UNE VALEUR DE FLUX SANGUIN

(84) Benannte Vertragsstaaten:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorität: **19.07.2016 DE 102016008821**

(43) Veröffentlichungstag der Anmeldung:
**29.05.2019 Patentblatt 2019/22**

(73) Patentinhaber: **Fresenius Medical Care
Deutschland GmbH
61352 Bad Homburg (DE)**

(72) Erfinder:
• **Dr. ZHANG, Wei
97464 Niederwerrn (DE)**
• **BARDORZ, Christoph
97228 Rottendorf (DE)**

(74) Vertreter: **Herrmann, Uwe
Lorenz Seidler Gossel
Rechtsanwälte Patentanwälte
Partnerschaft mbB
Widenmayerstraße 23
80538 München (DE)**

(56) Entgegenhaltungen:
**US-A1- 2002 151 804    US-A1- 2015 335 809**

**Beschreibung**

Die vorliegende Erfindung betrifft ein Dialysegerät mit einem extrakorporalen Blutkreislauf, in dem ein Dialysator, ein arterieller Drucksensor und eine Blutpumpe angeordnet sind, wobei das Dialysegerät des Weiteren über eine Kompensationseinrichtung verfügt, mittels derer der eingestellte Wert für den Blutfluss durch den extrakorporalen Kreislauf unter Verwendung des arteriellen Blutdrucks auf einen kompensierten Wert korrigierbar ist.

Aus dem Stand der Technik sind Dialysegeräte bekannt, die im extrakorporalen Kreislauf zwischen der arteriellen Nadel und der Blutpumpe einen arteriellen Drucksensor aufweisen. Dieser dient zur Erfassung des arteriellen Drucks bei einer Dialysebehandlung. Der Drucksensor ist über eine Druckableitung mit einer arteriellen Leitung des extrakorporalen Blutkreislaufes verbunden. Die Druckableitung weist üblicherweise einen Transducer-Protector (im Folgenden "TP") auf, der den Drucksensor vor Kontakt mit dem Patientenblut schützt. Dies wird meist durch eine Hydrophobmembran im TP erreicht.

Der TP dient somit zur Trennung der Blutseite von der Maschinenseite und verhindert die Kontamination der Maschine durch Blut, das durch den extrakorporalen Kreislauf fließt. Dies kann ansonsten zu Kreuzkontaminationen zwischen Patienten führen.

Der TP befindet sich üblicherweise in einer Druckableitung, d.h. z.B. in einem Schlauchstück, das den extrakorporalen Blutkreislauf, insbesondere dessen arterieller Abschnitt, mit dem arteriellen Drucksensor verbindet. Der TP enthält üblicherweise eine Hydrophobmembran, die das Blut von dem Drucksensor fern hält.

Bekannte Dialysegeräte verfügen des Weiteren über eine Kompensationseinrichtung, die unter Verwendung des Wertes des arteriellen Blutdrucks den von einem Nutzer des Dialysegerätes eingestellten Wert für den Blutfluss durch den extrakorporalen Kreislauf korrigiert, wobei der Behandlung der korrigierte Wert zugrunde gelegt wird. Diese Korrektur ist erforderlich, da aufgrund eines ovalisierten Schlauchquerschnitts stromauf der Blutpumpe der berechnete effektive Blutfluss ggf. nicht der eigentlichen, eingestellten Pumprate entspricht.

Der arterielle Druck im Blut wird somit benötigt, um einen Wert für einen kompensierten Blutfluss bereitzustellen. Dabei ist es aus dem Stand der Technik bekannt, den kompensierten, d.h. korrigierten Blutfluss $Q_b$ aus dem eingestellten Blutfluss $Q_{b,set}$ nach der folgenden Beziehung zu bestimmen.

$$Q_b = Q_{b,set}\,(\alpha + \beta * P_a) \tag{1}$$

Dabei gilt: $\alpha = 1{,}0$ und $\beta = 0{,}00057$; $Q$ in [ml/min], $P_a$ in [mmHg]

Wird die arterielle Druckableitung nicht angeschlossen, wird an dem arteriellen Drucksensor stets der Atmosphärendruck ($P_a = 0$ mmHg) gemessen. Dies bedeutet gemäß Gleichung (1), dass der angezeigte Blutfluss $Q_b$ gleich dem eingestellten Blutfluss $Q_{b,set}$ ist, das heißt eine Korrektur nicht stattfindet. Dies wiederum kann dazu führen, dass der angezeigte Wert für $Q_b$ den vom Bediener eingestellten Wert $Q_{b,set}$ um mehr als 11 % übersteigt. Dies hat zur Folge, dass die ärztliche Verschreibung bzw. das akkumulierte gereinigte Blutvolumen für eine Dialysebehandlung nicht erreicht wird, da die reale effektive Blutflussrate kleiner ist als die vom Nutzer eingestellte.

Aus der Praxis ist es bekannt, dass Behandlungen ohne eine angeschlossene Druckableitung und damit ohne angeschlossenen Drucksensor durchgeführt werden, was einen Missbrauch des Dialysegerätes darstellt. Gleichwohl soll auch für diesen Fall eine realistische Angabe des tatsächliches Blutflusses möglich sein.

Aus der WO 02/04044 A1 ist es bekannt, zur Detektion von arteriellen Einlaufproblemen während einer extrakorporalen Blutbehandlung die Amplitude der periodischen Schwankungen des Drucks, die auf die Umdrehungen der Blutpumpe zurückzuführen sind, in der venösen Blutleitung zu messen und mit einem Schwellenwert zu vergleichen. Auf Einlaufprobleme wird bei Überschreiten des Schwellenwertes geschlossen. Bei einer Dialysevorrichtung, die über ein Dialysierflüssigkeitssystem verfügt, kann anstelle des Drucks in der venösen Blutleitung auch der Druck im Dialysierflüssigkeitssystem überwacht werden. Eine arterielle Blutdruckmessung muss nicht erfolgen. Die US 2013/072846 A1 beschreibt ein Verfahren zum Bestimmen des Blutdrucks stromaufwärts der Blutpumpe durch Messung des Blutdrucks stromabwärts der Blutpumpe.

Aus den Druckschriften US2015/335809A1 und US 2002 / 151804A1 sind Dialysegeräte mit Erkennungsmitteln bekannt, mittels deren erkennbar ist, ob der arterielle Drucksensor an den extrakorporalen Blutkreislauf angeschlossen ist.

Der vorliegenden Erfindung liegt die Aufgabe zugrunde, ein Dialysegerät der eingangs genannten Art dahingehend weiterzubilden, dass auch bei nicht angeschlossenem arteriellen Drucksensor, insbesondere bei nicht angeschlossener arterieller Druckableitung die Angabe eines realistischen Wertes für den Blutfluss möglich ist.

Diese Aufgabe wird durch ein Dialysegerät mit den Merkmalen des Anspruchs 1 gelöst.

Danach ist vorgesehen, dass das Dialysegerät des Weiteren über Erkennungsmittel verfügt, die ausgebildet sind, zu erkennen, ob der arterielle Drucksensor an den extrakorporalen Blutkreislauf angeschlossen ist oder nicht, und dass das Dialysegerät eine Schätzeinheit aufweist, die ausgebildet ist, einen Wert für den arteriellen Blutdruck (Pa) zu schätzen, sofern durch die Erkennungsmittel erkannt wird, dass der arterielle Drucksensor nicht an den extrakorporalen Blutkreislauf angeschlossen ist, was beispielsweise dadurch bedingt sein kann, dass die arterielle Druckableitung nicht eingesetzt wurde.

Die vorliegende Erfindung basiert somit auf dem Gedanken, auch bei dem Fehlen des Anschlusses des Drucksensors an den extrakorporalen Kreislauf eine möglichst zuverlässige Kompensation bzw. Korrektur des eingestellten Blutflusses im extrakorporalen Kreislauf bereitstellen zu können. Dabei haben die Erkennungsmittel die Aufgabe, festzustellen, ob der arterielle Drucksensor angeschlossen ist oder nicht und die Schätzmittel haben die Aufgabe für den Fall, dass kein arterieller Drucksensor angeschlossen ist, einen Ersatzwert für den (nicht gemessenen) arteriellen Blutdruck im extrakorporalen Kreislauf bereitzustellen.

Unter einem "angeschlossenem Drucksensor" ist zu verstehen, dass der Drucksensor so mit dem extrakorporalen Kreislauf verbunden ist, dass dieser in der Lage ist, den Druck in dem extrakorporalen Kreislauf zu messen.

Das Nichtanschließen eines Drucksensors kann durch das Fehlen der Druckableitung bedingt sein, d.h. des Schlauch- bzw. Leitungsstücks, das den extrakorporalen Kreislauf mit dem Drucksensor verbindet und in dem sich üblicherweise der TP befindet.

Dieser kann dann verwendet werden, um die Kompensation des Blutflusses vorzunehmen. Der kompensierte, d.h. korrigierte Wert für den Blutfluss kann sodann der Behandlung zugrunde gelegt werden, angezeigt werden etc.

In einer denkbaren Ausgestaltung ist vorgesehen, dass die genannte Kompensationseinrichtung ausgebildet ist, den Wert für den kompensierten Blutfluss nach der obigen Gleichung (1) zu bestimmen. Bei Verwendung dieser Gleichung wird für $P_a$ nicht der gemessene, sondern der geschätzte arterielle Blutdruck verwendet.

Die Schätzeinheit kann ausgebildet sein, den arteriellen Blutdruck auf einen konstanten Wert, vorzugsweise auf den Wert von $P_a$ = -200 mmHg zu schätzen. Unabhängig von dem eingestellten Blutfluss kann z.B. nach Gleichung (1) mit einem konstanten Wert für $P_a$ ein Wert für $Q_b$ ermittelt werden. Der beispielhaft angegebene Wert von -200 mmHg entspricht in etwa dem Druckwert, der sich bei einem Blutfluss von $Q_{b,set}$ = 300 ml/min bei einer normalen Dialysebehandlung ergibt.

In einer weiteren Ausgestaltung der Erfindung ist die Schätzeinheit ausgebildet, den arteriellen Blutdruck in Abhängigkeit des nutzerseitig eingestellten Blutflusses $Q_{b,set}$ zu schätzen, wobei ein Speicher oder sonstiges Berechnungsmittel vorgesehen ist, in dem ein Zusammenhang zwischen dem arteriellen Blutdruck $P_a$ und dem nutzerseitig eingestellten Blutfluss $Q_{b,set}$ hinterlegt ist. Denkbar sind beispielsweise eine Kurve oder eine Tabelle für den Zusammenhang $P_a$ = f ($Q_{b,set}$). Auf der Grundlage dieser Werte, die in einer Software hinterlegt sein können, kann dann aus $Q_{b,set}$ der Wert für $P_a$ ermittelt werden und schließlich der Blutfluss z.B. nach Gleichung (1) berechnet werden.

Die Schätzeinheit kann des Weiteren derart ausgebildet sein, dass in den genannten Zusammenhang zwischen dem eingestellten Blutfluss und dem arteriellen Blutdruck weitere Parameter hineinspielen. In Betracht kommen beispielsweise eine Eigenschaft der verwendeten Kanüle, wie beispielsweise deren Außendurchmesser und/oder der Hämatokritwert HKT des Blutes.

Bei einem gegebenen arteriellen Blutschlauch kann somit der Zusammenhang zwischen dem geschätzten arteriellen Blutdruck und dem eingestellten Blutfluss noch durch die Dialysekanüle und/oder durch den Hämatokritwert des Blutes beeinflusst werden. Prinzipiell kann eine Kurvenschar bzw. Tabellen für die Funktion $P_a$ = f ($Q_{b,set}$) in Abhängigkeit der Dialysekanüle und des Hämatokrits experimentell ermittelt und in der Software hinterlegt werden. Der Typ der Dialysekanüle sowie der Hämatokritwert kann durch User-Angabe über ein User-Interface vor einer Dialysebehandlung festgelegt werden. Auch ist es denkbar, dass der Hämatokritwert während der Dialyse durch eine Dialysemaschine gemessen wird und dann der Messwert entsprechend verwendet wird.

Typische Dialysekanülen sind:

- 16 G mit 1,6 mm Außendurchmesser
- 17 G mit 1,5 mm Außendurchmesser
- 15 G mit 1,8 mm Außendurchmesser.

Typische Werte für den Hämatokritwert liegen im Bereich zwischen 27 und 42 %.

Für eine Realisierung dieser Ausführungsform der Erfindung ist es vorteilhaft, den Zusammenhang zwischen dem geschätzten arteriellen Blutdruck und dem eingestellten Blutfluss bei einer typischen Dialysekanüle z.B. bei einer 16 G Nadel und bei einem typischen Hämatokritwert, z.B. 35 % zu ermitteln und dann in der Software zu hinterlegen.

In einer weiteren Ausgestaltung der Erfindung ist vorgesehen, dass die Schätzeinheit ausgebildet ist, den arteriellen Blutdruck in Abhängigkeit des gemessenen venösen Blutdrucks im extrakorporalen Blutkreislauf zu schätzen.

Diese Ausführungsform der Erfindung wird anhand der einzigen Figur näher erläutert, die einen extrakorporalen Blutkreislauf eines Hämodialysegerätes gemäß der Erfindung darstellt.

Dabei kennzeichnet das Bezugszeichen 16 den Blutzugang, der als Fistel oder Shunt ausgebildet sein kann und die Bezugszeichen 15a und 15b die arterielle und die venöse Nadel.

Von der arteriellen Nadel 15a gelangt das Blut durch den Blutschlauch 14 über dier arterielle Druckableitung 1c zu dem arteriellen TP mit dem Bezugzeichen 1b, an den der arterielle Drucksensor 1a zur Messung des arteriellen Drucks $P_a$ angeschlossen ist. Stromabwärts davon befindet sich die Blutpumpe 3 und stromabwärts der Blutpumpe die Heparinpumpe 4. Zwischen der Heparinpumpe 4 und dem Dialysator befindet sich der arterielle Blasenfänger 5.

Die gestrichelte Linie in 1b stellt den Übergang von dem Schlauchsystem zum Dialysegerät dar. Dort wird somit die Druckableitung mit dem Drucksensor verbunden, z.B. mit einer Luer-Lock-Verbindung.

Der Dialysator ist mit dem Bezugzeichen 6 gekennzeichnet und ist durch eine semipermeable Membran 7, vorzugsweise durch ein Hohlfaserbündel, in eine Dialysatkammer und eine Blutkammer aufgeteilt, wobei an die Dialysatkammer der Dialysatzulauf 8 und der Dialysatablauf 9 angeschlossen sind.

Stromabwärts des Dialysators 6 befindet sich die venöse Tropfkammer 10 mit einem Leveldetektor 11. An die venöse Tropfkammer 10 angeschlossen ist die venöse Druckableitung 2c, die in Verbindung mit dem venösen Drucksensor mit dem Bezugzeichen 2a zur Messung des venösen Blutdruckes $P_v$ steht.In die venöse Druckableitung ist die venöse TP mit dem Bezugzeichen 2b eingebracht.

Stromabwärts der venösen Tropfkammer 10 befindet sich eine venöse Klemme 12, mittels derer der Blutschlauch absperrbar ist sowie der optische Detektor 13 zur Erkennung von Luftblasen im Blut.

Über die venöse Nadel 15b gelangt das Blut zurück in die Fistel bzw. in den Shunt 16.

Im Folgenden wird nun dargestellt, wie anhand des venösen Blutdruckes $P_v$, der z.B. in oder an der venösen Tropfkammer 10 gemessen werden kann, auf den arteriellen Blutdruck $P_a$ rückgeschlossen werden kann.

Analog des Ohm'schen Gesetzes gilt für die arterielle Seite des extrakorporalen Blutkreislaufes $Q_b = (P_{f,a} - P_a)/R_a$. Löst man diese Gleichung nach $P_a$ auf, ergibt sich:

$$P_a = P_{f,a} - Q_b * R_a \qquad\qquad (2')$$

Auf der venösen Seite des extrakorporalen Blutkreislaufes ergibt sich $Q_b - Q_{UF} = (P_v - P_{f,v})/R_v$.

Löst man diese Gleichung nach dem venösen Druck auf, ergibt sich:

$$P_v = (Q_b - Q_{UF}) \overset{\cdot}{*} R_v + P_{f,v} \qquad\qquad (3')$$

Addiert man die Gleichungen (2') und (3') ergibt sich:

$$P_a + P_v = Q_b * (R_v - R_a) + (P_{f,a} + P_{f,v}) - Q_{UF} * R_v \qquad\qquad (4')$$

Im Rahmen der vorliegenden Erfindung sind: $P_f$ der Fisteldruck, $P_{f,a}$ der Fisteldruck an der Stelle der arteriellen Dialysenadel, $P_{t,v}$ der Fisteldruck an der Stelle der venösen Dialysenadel, $P_a$ der arterielle Druck vor der Blutpumpe, $P_v$ der venöse Druck an der venösen Tropfkammer, $P_{v,0}$ der gemessene venöse Druck beim Stopp der Blutpumpe und bei geöffneter venösen Klemme, $Q_{b,set}$ der eingestellte Blutfluss, $Q_b$ der kompensierte Blutfluss, $Q_{UF}$ die Ultrafiltrationsrate, $R_a$ der Strömungswiderstand zwischen der arteriellen Druckableitung 1c und der Spitze der arteriellen Nadel, $R_v$ der Strömungswiderstand zwischen der venösen Tropfkammer und der Spitze der venösen Nadel und MAP der mittlere gemessene arterielle Blutdruck (mean arterial blood pressure) des Dialysepatienten.

Die Länge des arteriellen Schlauchabschnittes zwischen der arteriellen Druckableitung und dem arteriellen Nadelkonnektor ist nahezu identisch zur Länge des venösen Schlauchabschnittes zwischen der venösen Tropfkammer und dem venösen Nadelkonnektor. Diese Länge kann beispielsweise ca. 190 cm betragen. Unter Berücksichtigung der Tatsache, dass in einer Routinedialyse arterielle und venöse Nadeln eines gleichen Typs, z.B. 16 G verwendet werden, sind die Strömungswiderstände der oben genannten Schlauchabschnitte auch nahezu identisch, das heißt es gilt in Gleichung (4') $R_a = R_v$. Daraus ergibt sich:

$$P_a = (P_{f,a} + P_{f,v}) - Q_{UF} * R_v - P_v \qquad\qquad (5')$$

Die Ultrafiltrationsrate $Q_{UF}$ in der Dialyse ist relativ klein. Vernachlässigt man diesen Wert oder misst man $P_a$ bei ausgeschalteter Ultrafiltration ergibt sich aus Gleichung (5') die folgende Beziehung:

$$P_a = (P_{f,a} + P_{f,v}) - P_v \qquad\qquad (6')$$

Die Drucke im Shunt bzw. in der Fistel können wie folgt abgeschätzt werden:

Für den Fall der Fistel gilt:

$$P_{f,a} = 0{,}20 * MAP$$

$$P_{f,v} = 0{,}15 * MAP$$

$$P_{f,a} = 1{,}33 * P_{f,v}$$

Für den Shunt gelten folgende Beziehungen:

$$P_{f,a} = 0{,}55 * MAP$$

$$P_{f,v} = 0{,}35 * MAP$$

$$P_{f,a} = 1{,}57 * P_{f,v}$$

Daraus ergibt sich aus Gleichung (6') im Fistelfall

$$P_a = 0{,}35 * MAP - P_v \qquad\qquad (7')$$

sowie

$$P_a = 2{,}33 * P_{f,v} - P_v \qquad\qquad (8')$$

Aus Gleichung (6') ergibt sich im Shunt-Fall

$$P_a = 0{,}90 * MAP - P_v \qquad\qquad (9')$$

sowie

$$P_a = 2{,}57 * P_{f,v} - P_v \qquad\qquad (10')$$

Bei der Hämodialyse ist es verpflichtend, den Blutdruck eines Patienten zu messen. Der mittlere arterielle Blutdruck MAP in den Gleichungen (7') und (9') ist deshalb bekannt. Dieser MAP-Wert kann entweder durch einen in die Dialysemaschine integrierten Blutdruckmonitor (BPM) automatisch ermittelt werden oder per User Interface manuell eingegeben werden. In diesen Fällen kann der gesuchte Wert $P_a$ bei gemessenen $P_v$ nach Gleichung (7') oder nach Gleichung (9') ermittelt werden.

Wie dies aus den Gleichungen (8') und (10') hervorgeht, wird in diesen Fällen zur Bestimmung von $P_a$ der Wert $P_{f,v}$ benötigt, der in einer weiteren bevorzugten Ausgestaltung durch die Beziehung $P_{f,v} = P_{v0} + 14,72$ ermittelt werden kann.

Diese Beziehung ergibt sich wie folgt:

Nach der Verbindung des Patienten mit dem extrakorporalen Blutkreislauf wird die Blutpumpe gestartet und das Patientenblut in den extrakorporalen Blutkreislauf gefördert. Sobald der optische Detektor das Blut an der venösen Tropfkammer erkennt, wird die Blutpumpe gestoppt und die venöse Klemme 12 geschlossen. Anschließend wird der Anwender gefragt, ob der Dialysebehandlung gestartet werden soll. Wird in diesem Fall die venöse Klemme kurz geöffnet, wird der venöse Druck als $P_{v0}$ gemessen.

Unter Berücksichtigung des hydrostatischen Druckes zwischen dem Fistelzugang am Patientenarm und der venösen Druckableitung 2c errechnet sich der Wert für $P_{f,v}$ nach

$$P_{f,v} = P_{v0} + r * g * H \qquad (11')$$

Dabei ist r die Wasserdichte und beträgt 1000 kg/m3, H ist der Höhenunterschied zwischen dem Fistelzugang und der venösen Druckableitung 2c, z.B. 20 cm und g ist die Erdbeschleunigung (9,81 m/s$^2$). Aus den Gleichungen (8'), (10') und (11') ergeben sich für die Fistel

$$P_a = 2,33 * (P_{v0} + r * g * H) - P_v \qquad (12')$$

und für den Shunt

$$P_a = 2,57 * (P_{v0} + r * g * H) - P_v \qquad (13')$$

Mit einer Höhendifferenz von 0,2 m ergibt sich daraus:

$$r * g * H = 1000 * 9,81 * 0,2 = 14,72 \text{ mmHg.}$$

Setzt man diesen Wert in Gleichung (11') ein, ergibt sich die genannte Beziehung, nämlich

$$P_{f,v} = P_{v0} + 14,72 \text{ [mmHg]} \qquad (14')$$

Bei Kenntnis dieses Wertes sowie des venösen Blutdrucks lässt sich aus den Gleichungen (8') und (10') der Wert $P_a$ bestimmen.

In einer weiteren Ausführungsform der Erfindung ist vorgesehen, dass die Erkennungsmittel ausgebildet sind, das mittels des arteriellen Drucksensors erfasste Signal zu ermitteln, eine laufenden Mittelwertbildung und Varianzberechnung über einen bestimmten Zeitraum, z.B. eine Minute, durchzuführen und anhand der Auswertung von Mittelwert und Varianzwert auf das Vorhandensein oder Fehlen eines angeschlossenen arteriellen Drucksensors zu schließen. Das Fehlen dieses Anschlusses kann beispielsweise darauf zurückgeführt werden, dass die arterielle Druckableitung (mit dem ggf. darin befindlichen TP) nicht eingesetzt ist, so dass der arterielle Drucksensor nicht den Druck in dem extrakorporalen Blutkreislauf messen kann.

In einer alternativen oder zusätzlich anwendbaren Ausführungsform ist vorgesehen, dass die Erkennungsmittel ausgebildet sind, das mittels des arteriellen Drucksensors erfasste Drucksignal zu ermitteln, die Blutpumpe zu starten und zu stoppen, während das Blutschlauchsystem gefüllt ist, die Signaländerung des arteriellen Blutdrucks zu bestimmen und zu bewerten und basierend darauf auf das Fehlen oder Vorhandensein der arteriellen Druckableitung bzw. des Anschlusses des arteriellen Drucksensors zu schließen.

Wird kein angeschlossener Drucksensor erkannt, wird die Blutflusskompensation mithilfe der oben beschriebenen Verfahren vorgenommen, wobei vorzugsweise Gleichung (1) verwendet wird, wobei für $P_a$ ein erfindungsgemäß geschätzter Wert herangezogen wird.

Andernfalls wird der gemessene Wert für $P_a$ verwendet.

Die vorliegende Offenbarung betrifft des Weiteren ein nicht beanspruchtes Verfahren zur Korrektur eines eingestellten Wertes für den Blutfluss unter Verwendung des arteriellen Blutdruckes auf einen kompensierten Wert in einem Dialysegerät mit einem extrakorporalen Blutkreislauf, in dem ein Dialysator eine Blutpumpe sowie ein arterieller Drucksensor angeordnet sind, wobei erfasst wird, ob der Drucksensor an dem extrakorporalen Kreislauf angeschlossen ist oder nicht und wobei ein Wert für den arteriellen Blutdruck geschätzt wird, sofern festgestellt wird, dass dies nicht der Fall ist. Dabei kann der Wert für den kompensierten Blutfluss nach der obigen Gleichung (1) bestimmt werden.

Der arterielle Blutdruck kann auf einen konstanten Wert, wie beispielsweise auf den Wert -200 mmHg geschätzt werden. Auch ist es möglich den arteriellen Blutdruck in Abhängigkeit des nutzerseitig eingestellten Blutflusses zu schätzen, wobei softwaremäßig bzw. in einem Speicher oder durch eine Berechnungseinheit ein entsprechender Zusammenhang zwischen dem arteriellen Blutdruck und dem eingestellten Blutfluss hinterlegt ist. Dabei können weitere Parameter eine Rolle spielen, insbesondere die Art der verwendeten Dialysekanüle und insbesondere deren Außendurchmesser und/oder der gemessene oder eingegebene Hämatokritwert des Blutes.

Auch ist es denkbar, wie oben ausgeführt, dass der arterielle Blutdruck in Abhängigkeit des venösen Blutdrucks im extrakorporalen Kreislauf geschätzt wird.

Dabei ist es denkbar, dass folgende Beziehungen zur Anwendung kommen:

$$P_a = 0{,}35 * MAP - P_v,$$

$$P_a = 2{,}33 * P_{f,v} - P_v$$

bzw.

$$P_a = 0{,}90 * MAP - P_v,$$

$$P_a = 2{,}57 * P_{f,v} - P_v$$

Zur Herleitung dieser Beziehungen verweisen wir auf die obigen Ausführungen.

In einer weiteren Ausgestaltung des Verfahrens ist vorgesehen, dass der Wert $P_{f,v}$ berechnet wird, nachdem Blut in den extrakorporalen Kreislauf bis zum venösen Drucksensor eingeleitet wird, dann die venöse Druckklemme geöffnet wird und sodann bei stehender Blutpumpe der venöse Blutdruck $P_{v0}$ gemessen wird. Dabei ergibt sich der Wert von $P_{f,v}$ aus der Beziehung $P_{f,v} = P_{v0} + 14{,}72$. Zur Ermittlung dieser Gleichung verweisen wir ebenfalls auf die obigen Ausführungen.

In einer weiteren Ausgestaltung des Verfahrens ist vorgesehen, dass zur Erkennung des Vorhandenseins oder Fehlens des Anschlusses des arteriellen Drucksensors das durch den arteriellen Drucksensor erfasste Drucksignal ermittelt wird und anhand einer laufenden Mittelwertbildung und Varianzberechnung über einen bestimmten Zeitraum geprüft wird, ob der Drucksensor anschgeschlossen ist oder nicht. Auch ist es denkbar, das durch den arteriellen Drucksensor erfasste Drucksignal zu ermitteln, während die Blutpumpe gestartet und wieder gestoppt wird. Das Drucksignal gibt Rückschlüsse auf das Vorhandensein oder Fehlen des Anschlusses des arteriellen Drucksensors bzw. der arteriellen Druckableitung.

Grundsätzlich ist es denkbar, dass die Erkennung, ob der Drucksensor angeschlossen ist, darauf basiert, dass der Drucksensor keinerlei Druckschwankungen misst, obwohl die Blutpumpe läuft, oder dass mit der Blutpumpe ein Druckpuls aufgegeben wird, der am Drucksensor nicht gemessen wird.

Weitere Einzelheiten und Vorteile der Erfindung werden anhand eines in der Zeichnung und im Folgenden beschriebenen Ausführungsbeispiels näher erläutert.

Die einzige Figur zeigt einen extrakorporalen Blutkreislauf, der über eine venöse Nadel und eine arterielle Nadel an eine Fistel bzw. an einen Shunt des Patienten angeschlossen ist.

Zunächst wird über ein in der Zeichnung nicht dargestelltes User Interface des Dialysegerätes, in dem der extrakorporale Blutkreislauf gemäß der Figur eingesetzt ist, eingegeben, ob es sich bei dem Blutzugang um eine Fistel oder um einen Shunt handelt. Sodann erfolgt die Eingabe des gemessenen MAP, das heißt des mittleren Blutdrucks des Patienten.

Ab dem Zeitpunkt der Bluterkennung durch den optischen Detektor 13 werden z.B. alle zwei Minuten die Werte

für $P_a$ und $P_v$ segmentenweise zur Erkennung eines nicht angeschlossenen Drucksensors bzw. Druckableitung ausgewertet. Wird erkannt, dass beide Drucksensoren bzw. Druckableitungen angeschlossen sind, das heißt der arterielle und der venöse, wird der Blutfluss nach Gleichung (1) kompensiert, wobei für $P_a$ der gemessene arterielle Blutdruck herangezogen wird.

Wird festgestellt, dass keiner der Drucksensoren, das heißt weder der venöse noch der arterielle angeschlossen sind, wird eine Warnung ausgegeben, die den Anwender auffordert, beide Drucksensoren anzuschließen. Wird festgestellt, dass der venöse Drucksensor angeschlossen ist, der arterielle Drucksensor jedoch nicht, wird eine Warnung ausgegeben, die den Anwender auffordert, auch den arteriellen Drucksensor anzuschließen. Erfolgt dies nicht, läuft das Verfahren wie folgt weiter.

Es wird bei laufender Blutpumpe während der normalen Dialysebehandlung der venöse Druck des Blutes im extrakorporalen Kreislauf, das heißt $P_v$ gemessen. Sodann erfolgt die Bildung eines laufenden Mittelwertes von $P_v$.

Nach Gleichung (7') oder nach Gleichung (9') erfolgt die Berechnung, das heißt die Schätzung von $P_a$ für die Fistel oder für den Shunt. Basierend auf diesen Werten kann dann mittels Gleichung (1) der Wert für den kompensierten Blutfluss, das heißt $Q_b$ bestimmt werden.

Dabei ist es denkbar, dass dieser Wert begrenzt wird auf den Bereich

$$0{,}85\ Q_{b,set} \leq Q_b \leq Q_{b,set}$$

Vorzugsweise wird das Kompensationsverfahren ausschließlich für die aktive Dialysephase, das heißt wenn Blut im System erkannt ist, die Blutpumpe läuft und die Dialyse alarmfrei ist, durchgeführt. Eine Kompensation in der Phase des Vorbereitens und der Reinfusion ist zwar denkbar, prinzipiell jedoch nicht notwendig.

Abschließend wird darauf hingewiesen, dass der im Rahmen der Erfindung verwendete Begriff "ein" oder "eine" ein einziges der betreffen Elemente, jedoch auch eine Mehrzahl dieser Elemente mit umfasst, d.h. nicht zwingend mit "ein/eine einzige(s)" gleichzusetzen ist.

**Patentansprüche**

1. Dialysegerät mit einem extrakorporalen Blutkreislauf (1), in dem ein Dialysator (6), eine Blutpumpe (3) und ein arterieller Drucksensor (1a) angeordnet sind, wobei das Dialysegerät des Weiteren über eine Kompensationseinrichtung verfügt, mittels derer der eingestellte Wert für den Blutfluss ($Q_{b,set}$) durch den extrakorporalen Kreislauf (1) unter Verwendung des arteriellen Blutdrucks ($P_a$) auf einen kompensierten Wert ($Q_b$) korrigierbar ist, wobei das Dialysegerät des Weiteren über Erkennungsmittel verfügt, die ausgebildet sind, zu erkennen, ob der arterielle Drucksensor (1a) an den extrakorporalen Blutkreislauf (1) angeschlossen ist oder nicht,
**dadurch gekennzeichnet,**
**dass** das Dialysegerät eine Schätzeinheit aufweist, die ausgebildet ist, einen Wert für den arteriellen Blutdruck ($P_a$) zu schätzen, sofern durch die Erkennungsmittel erkannt wird, dass der arterielle Drucksensor (1a) nicht an den extrakorporalen Blutkreislauf (1) angeschlossen ist.

2. Dialysegerät nach Anspruch 1, **dadurch gekennzeichnet, dass** das Dialysegerät derart ausgebildet ist, das eine Druckableitung (1c) anschließbar ist, die im angeschlossenen Zustand den arteriellen Drucksensor (1a) mit dem extrakorporalen Blutkreislauf (1) verbindet, und dass die Erkennungsmittel ausgebildet sind, zu erkennen, ob die Druckableitung (1c) angeschlossen ist oder nicht.

3. Dialysegerät nach Anspruch 1 oder 2, **dadurch gekennzeichnet, dass** die Kompensationseinrichtung ausgebildet ist, den Wert für den kompensierten Blutfluss ($Q_b$) in [ml/min] nach der Gleichung (1) zu bestimmen:

$$Q_b = Q_{b,set}\ (\alpha + \beta\ {}^* P_a) \qquad (1),$$

wobei $\alpha$ den Wert 1,0 und $\beta$ den Wert 0,00057 annimmt und $P_a$ als Wert in [mmHg] verwendet wird.

4. Dialysegerät nach einem der vorhergehenden Ansprüche, **dadurch gekennzeichnet, dass** die Schätzeinheit ausgebildet ist, den arteriellen Blutdruck ($P_a$) auf einen konstanten Wert, vorzugweise auf den Wert - 200 mmHg zu schätzen und/oder dass die Schätzeeinheit ausgebildet ist, den arteriellen Blutdruck ($P_a$) in Abhängigkeit des nutzerseitig eingestellten Blutflusses ($Q_{b,set}$) zu schätzen, wobei ein Speicher oder eine Berechnungseinheit vorgesehen ist, in dem/der ein Zusammenhang zwischen dem arteriellen Blutdruck ($P_a$) und dem nutzerseitig eingestellten Blutfluss ($Q_{b,set}$) hinterlegt ist, wobei vorzugsweise vorgesehen ist, dass die Schätzeinheit derart ausgebildet ist, dass in den genannten Zusammenhang als weiterer Parameter eine Eigenschaft der verwendeten Dialysekanüle und/oder der Hämatokritwert (HKT) des Blutes eingeht.

5. Dialysegerät nach einem der vorhergehenden Ansprüche, **dadurch gekennzeichnet, dass** die Schätzeinheit ausgebildet ist, den arteriellen Blutdruck ($P_a$) in Abhängigkeit des venösen Blutdrucks ($P_v$) im extrakorporalen Kreislauf zu schätzen, wobei vorzugsweise vorgesehen ist, dass die Schätzeinheit ausgebildet ist, den arteriellen Blutdruck ($P_a$) in [mmHg] nach einer der Beziehungen zu ermitteln ($P_v$: venöser Blutdruck im extrakorporalen Kreislauf; MAP: mittlerer Blutdruck; $P_{f,v}$: Fisteldruck/Shuntdruck an der Stelle der venösen Dialysenadel)

$$P_a = 0{,}35 * MAP - P_v \qquad (2)$$

$$P_a = 2{,}33 * P_{f,v} - P_v \qquad (3)$$

$$P_a = 0{,}90 * MAP - P_v \qquad (4)$$

$$P_a = 2{,}57 * P_{f,v} - P_v \qquad (5),$$

wobei die Gleichungen (2) und (3) für die Verwendung einer Fistel und die Gleichungen (4) und (5) für die Verwendung eines Shunts zur Anwendung kommen.

6. Dialysegerät nach Anspruch 5, **dadurch gekennzeichnet, dass** das Dialysegerät eine Berechnungseinheit zur Bestimmung von $P_{f,v}$ aufweist und dass diese Berechnungseinheit ausgebildet ist, nach dem Einleiten von Blut in den extrakorporalen Kreislauf bis zum venösen Drucksensor eine venöse Druckklemme (12) zu öffnen und dann den venösen Blutdruck ($P_{v0}$) bei stehender Blutpumpe zu messen, und dass die Berechnungseinheit ausgebildet ist, den Wert für $P_{f,v}$ in [mmHg] wie folgt zu bestimmen:

$$P_{f,v} = P_{v0} + 14{,}72 \qquad (6)$$

7. Dialysegerät nach einem der vorhergehehen Ansprüche, **dadurch gekennzeichnet, dass** die Erkennungsmittel ausgebildet sind, das mittels des arteriellen Drucksensors (1a) erfasste Drucksignal zu ermitteln, eine laufende Mittelwertbildung und Varianzberechnung über einen bestimmten Zeitraum durchzuführen und anhand der Auswertung von Mittelwert und Varianzwert auf das Vorhandensein oder Fehlen des Anschlusses des arteriellen Drucksensors (1a) an den extrakorporalen Blutkreislauf (1) zu schließen.

8. Dialysegerät nach einem der vorhergehenden Ansprüche, dadurch gekennzeichnet, dass die Erkennungsmittel ausgebildet sind, das mittels des arteriellen Drucksensors (1a) erfasste Drucksignal zu ermitteln, die Blutpumpe zu starten und wieder zu stoppen, die Änderung des arteriellen Blutdrucks ($P_a$) zu bestimmen und basierend darauf auf das Vorhandensein oder Fehlen des Anschlusses des arteriellen Drucksensors (1a) an den extrakorporalen Blutkreislauf (1) zu schließen.

**Claims**

1. Dialysis machine having an extracorporeal blood circuit (1) in which a dialyzer (6), a blood pump (3) and an arterial pressure sensor (1a) are arranged, wherein the dialysis machine furthermore has a compensation device by means of which the value set for the blood flow ($Q_{b,set}$) can be corrected to a compensated value ($Q_b$) by the extracorporeal circuit (1) using the arterial blood pressure ($P_a$), wherein the dialysis machine furthermore has detection means which are configured to recognize whether the arterial pressure sensor (1a) is connected to the extracorporeal blood circuit (1) or not,
**characterized in that**
the dialysis machine has an estimator unit which is configured to estimate a value for the arterial blood pressure ($P_a$) if it is recognized by the recognition means that the arterial pressure sensor (1a) is not connected to the extracorporeal blood circuit (1).

2. Dialysis machine in accordance with claim 1, **characterized in that** the dialysis machine is configured such that a pressure return line (1c) can be connected which connects the arterial pressure sensor (1a) to the extracorporeal blood circuit (1) in the connected state; and **in that** the recognition means are configured to recognize whether the pressure return line (1c) is connected or not.

3. Dialysis machine in accordance with claim 1 or claim 2, **characterized in that** the compensation device is configured to determine the value for the compensated blood flow ($Q_b$) in [ml/min] in accordance with equation (1):

$$Q_b = Q_{b,set} \left( \alpha + \beta * P_a \right) \qquad (1),$$

where $\alpha$ adopts the value 1.0 and $\beta$ adopts the value 0.00057 and $P_a$ is used as a value in [mmHg].

4. Dialysis machine in accordance with one of the preceding claims, **characterized in that** the estimator unit is configured to estimate the arterial blood pressure ($P_a$) to a constant value, preferably to the value -200 mmHg; and/or **in that** the estimator unit is configured to estimate the arterial blood pressure ($P_a$) in dependence on the blood flow ($Q_{b,set}$) set by the user, wherein a memory or a calculation unit is provided in which a relationship is stored between the arterial blood pressure ($P_a$) and the blood flow ($Q_{b,set}$) set by the user, with provision preferably being made that the estimator unit is configured such that a property of the used dialysis cannula and/or the hematocrit value (HKT) of the blood is taken into the named relationship as a further parameter.

5. Dialysis machine in accordance with one of the preceding claims, **characterized in that** the estimator unit is configured to estimate the arterial blood pressure ($P_a$) in dependence on the venous blood pressure ($P_v$) in the extracorporeal blood circuit, with provision preferably being made that the estimator unit is configured to determine the arterial blood pressure ($P_a$) in [mmHg] in accordance with one of the relationships ($P_v$: venous blood pressure in the extracorporeal circuit; MAP: mean blood pressure; $P_{f,v}$ fistula pressure/shunt pressure at the point of the venous dialysis needle)

$$P_a = 0.35 * MAP - P_v \qquad (2)$$

$$P_a = 2.33 * P_{f,v} - P_v \qquad (3)$$

$$P_a = 0.90 * MAP - P_v \qquad (4)$$

$$P_a = 2.57 * P_{f,v} - P_v \qquad (5),$$

with equations (2) and (3) being applied for the use of a fistula and equations (4) and (5) being applied for the use of a shunt.

6. Dialysis machine in accordance with claim 5, **characterized in that** the dialysis machine has a computation unit for determining $P_{f,v}$; and **in that** this computation unit is configured to open a venous pressure clamp (12) after the introduction of blood into the extracorporeal circuit up to the venous pressure sensor and then to measure the venous blood pressure ($P_{v0}$) with an idling blood pump; and **in that** the computation unit is configured to determine the value for $P_{f,v}$ in [mmHg] as follows:

$$P_{f,v} = P_{v0} + 14.72 \qquad (6)$$

7. Dialysis machine in accordance with one of the preceding claims, **characterized in that** the recognition means are configured to determine the pressure signal detected by means of the arterial pressure sensor (1a), to carry out an ongoing mean value formation and variance calculation over a specific period in time and to draw a conclusion on the presence or absence of the connection of the arterial pressure sensor (1a) to the extracorporeal blood circuit (1) with reference to the evaluation of the mean value and the variance value.

8. Dialysis machine in accordance with one of the preceding claims, **characterized in that** the recognition means are configured to determine the pressure signal detected by means of the arterial pressure sensor (1a), to start the blood pump and to stop it again, to determine the change of the arterial blood pressure ($P_a$) and, based on this, to draw a conclusion on the presence or absence of the connection of the arterial pressure sensor (1a) to the extracorporeal blood circuit (1).

**Revendications**

1. Appareil de dialyse avec un circuit de sang extracorporel (1), dans lequel sont disposés un dialyseur (6), une pompe à sang (3) et un capteur de pression artérielle (1a), dans lequel l'appareil de dialyse dispose par ailleurs d'un dispositif de compensation, au moyen duquel la valeur réglée pour le flux sanguin ($Q_{b,set}$) à travers le circuit extracorporel (1) peut être corrigée sur une valeur compensée ($Q_b$) en utilisant la pression sanguine artérielle ($P_a$), dans lequel l'appareil de dialyse dispose par ailleurs de moyens d'identification qui sont réalisés pour identifier si le capteur de pression artérielle (1a) est raccordé ou non au circuit de sang extracorporel (1),
**caractérisé en ce**
**que** l'appareil de dialyse présente une unité d'évaluation qui est réalisée pour évaluer une valeur pour la pression sanguine artérielle ($P_a$) dans la mesure où les moyens d'identification ont pu identifier que le capteur de pression artérielle (1a) n'est pas raccordé au circuit de sang extracorporel (1).

2. Appareil de dialyse selon la revendication 1, **caractérisé en ce que** l'appareil de dialyse est réalisé de telle manière qu'une dérivation de pression (1c) peut être raccordée, qui relie, dans l'état raccordé, le capteur de pression artérielle (1a) au circuit de sang extracorporel (1), et que les moyens d'identification sont réalisés pour identifier si la dérivation de pression (1c) est raccordée ou non.

3. Appareil de dialyse selon la revendication 1 ou 2, **caractérisé en ce que** le dispositif de compensation est réalisé pour définir la valeur pour le flux sanguin compensé ($Q_b$) en [ml/min] selon l'équation (1) :

$$Q_b = Q_{b,set} (\alpha + \beta * P_a) \qquad (1),$$

dans lequel $\alpha$ prend la valeur 1,0 et $\beta$ prend la valeur 0,00057 et $P_a$ est utilisée en tant que valeur en [mmHg].

4. Appareil de dialyse selon l'une quelconque des revendications précédentes, **caractérisé en ce que** l'unité d'évaluation est réalisée pour évaluer la pression sanguine artérielle ($P_a$) sur une valeur constante, de préférence sur la valeur - 200 mmHg, et/ou que l'unité d'évaluation est réalisée pour évaluer la pression sanguine artérielle ($P_a$) en fonction du flux sanguin ($Q_{b,set}$) réglé du côté de l'utilisateur, dans lequel un système de stockage ou une unité de calcul est prévu ou prévue, dans lequel/laquelle un lien entre la pression sanguine artérielle ($P_a$) et le flux sanguin ($Q_{b,set}$) réglé du côté de l'utilisateur est enregistré, dans lequel de préférence il est prévu que l'unité d'évaluation est réalisée de telle manière qu'une propriété de la canule de dialyse utilisée et/ou le taux d'hématocrite (HCT) du sang sont intégrés dans ledit lien en tant qu'autre paramètre.

5. Appareil de dialyse selon l'une quelconque des revendications précédentes, **caractérisé en ce que** l'unité d'évaluation est réalisée pour évaluer la pression sanguine artérielle ($P_a$) en fonction de la pression sanguine veineuse ($P_v$) dans le circuit extracorporel, dans lequel il est prévu de préférence que l'unité d'évaluation est réalisée pour déterminer la pression sanguine artérielle ($P_a$) en [mmHg] selon une des relations ($P_v$ : pression sanguine veineuse dans le circuit extracorporel ; MAP : pression sanguine moyenne ; $P_{f,v}$: pression dans la fistule/pression dans le shunt à l'emplacement de l'aiguille de dialyse veineuse)

$$P_a = 0.35 * MAP - P_v \qquad (2)$$

$$P_a = 2.33 * P_{f,v} - P_v \qquad (3)$$

$$P_a = 0.90 * MAP - P_v \qquad (4)$$

$$P_a = 2.57 * P_{f,v} - P_v \qquad (5),$$

dans lequel les équations (2) et (3) sont appliquées pour l'utilisation d'une fistule et les équations (4) et (5) sont appliquées pour l'utilisation d'un shunt.

6. Appareil de dialyse selon la revendication 5, **caractérisé en ce que** l'appareil de dialyse présente une unité de calcul pour définir $P_{f,v}$, et que ladite unité de calcul est réalisée pour ouvrir, après l'introduction de sang dans le circuit extracorporel jusqu'au capteur de pression veineuse, un clamp veineux (12) puis pour mesurer la pression sanguine veineuse ($P_{v0}$) lors de l'arrêt de la pompe à sang, et que l'unité de calcul est réalisée pour définir la valeur pour $P_{f,v}$ en [mmHg] comme suit :

$$P_{f,v} = P_{v0} + 14.72 \qquad (6)$$

7. Appareil de dialyse selon l'une quelconque des revendications précédentes, **caractérisé en ce que** les moyens d'identification sont réalisés pour déterminer le signal de pression détecté au moyen du capteur de pression artérielle (1a), pour mettre en œuvre en continu une obtention de valeur moyenne et un calcul de variance sur une période définie et pour conclure, à l'aide de l'estimation de la valeur moyenne et de la valeur de variance, à la présence ou à l'absence du raccordement du capteur de pression artérielle (1a) au circuit de sang extracorporel (1).

8. Appareil de dialyse selon l'une quelconque des revendications précédentes, **caractérisé en ce que** les moyens d'identification sont réalisés pour déterminer le signal de pression détecté au moyen du capteur de pression artérielle (1a), pour démarrer la pompe à sang puis l'arrêter à nouveau, pour définir la variation de la pression sanguine artérielle ($P_a$) et pour conclure sur cette base à la présence ou à l'absence du raccordement du capteur de pression artérielle (1a) au circuit de sang extracorporel (1).

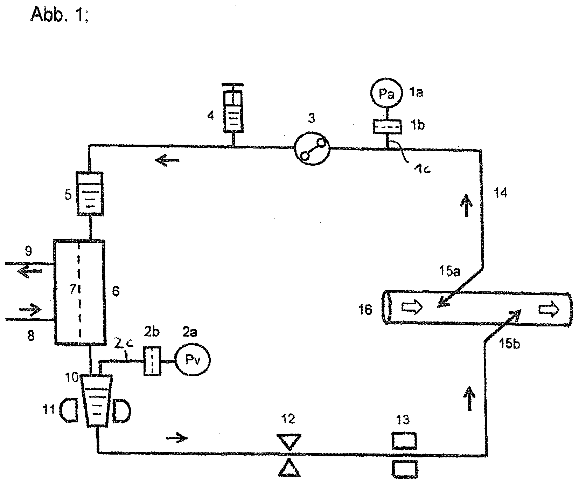

Abb. 1:

EP 3 487 556 B1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 0204044 A1
- US 2013072846 A1

- US 2015335809 A1
- US 2002151804 A1